# EUROPEAN PATENT SPECIFICATION

(11) **EP 1 239 742 B1**
(45) Date of publication and mention of the grant of the patent: **07.09.2005**
(21) Application number: 00991353.4
(22) Date of filing: 22.12.2000
(51) Int. Cl.: A23L 1/08, A23L 1/10, A61K 35/78, A61K 35/64, A23L 1/172, A61P 1/00

(54) **Honey based food product for relief of gastroesophageal reflux disease and stomach complaints and compressed product comprising said composition**
Nahrungsmittel auf Basis von Honig zur Verminderung von Gastroösophagealen Reflux-Krankheit und Störungen im Magenbereich sowie Presserzeugnisse aus dieser Zusammensetzung
Composition à base de miel destinée à soulager le reflux gastro-oesophagien ainsi que les maux d'estomac et produit comprimé comprenant ladite composition.

(30) Priority: 23.12.1999 NL 1013943
(43) Date of publication of application: 18.09.2002
(73) Proprietor: Triticum Holding B.V., 6211 TE Maastricht (NL); C.N.C.I. BVBA, 8490 Varsenare (BE); Bees Best Natural Products LLC, Ft. Lauderdale, FL 33316 (US)
(72) Inventor: POSTMES, Theodore, Justin, Louis, Joseph, NL-6211 TE Maastricht (NL)
(74) Representative: Valkonet, Rutger
(86) International application number: PCT/NL2000/000952
(87) International publication number: WO 2001/047373

(56) References cited:
- EP-A- 0 779 038
- WO-A-89/07399
- DE-A- 4 322 421
- FR-A- 2 405 658
- GB-A- 821 883
- GB-A- 1 571 251
- DATABASE WPI Week 199514 Derwent Publications Ltd., London, GB; AN 1995-100745 XP002141226 & HU 66 713 A (BOZSIK), 28 December 1994 (1994-12-28)
- DATABASE WPI Week 199127 Derwent Publications Ltd., London, GB; AN 1991-197903 XP002141227 & JP 03 123460 A (YAMAICHIYA)

## Description

The present invention generally relates to a honey-based composition for gastroesophageal reflux disease (GERD) and stomach complaints, and to a compressed product comprising said composition.

Indigestion, as well as heartburn, occurs frequently. Stomach pain and severe heartburn are, if they only occur from time to time, a normal deviance of the digestion physiology. Overweight, over eating, over consumption of alcohol, worry and stress are correlating factors to the appearance of disturbances in the digestion (dyspepsia). The over-production of acid is a characteristic of this disturbance, which can be remedied by reducing the production of acid or by neutralizing the stomach acid with antacids (e.g., with calcium carbonate and magnesium carbonate such as PENNIES (brand)). Ingestion of currently available pharmaceutical products for dealing with digestive disorders is accompanied by one or more side effects. In order to fight dyspepsia, one has foremost to change something in one's lifestyle and, in addition, the emphasis should be on good nourishment.

Products, for the relief of stomach and intestine complaints having honey as a component are found, for example in Chinese patent publications CN 1189362 and CN 1143514. Such products contain besides honey numerous components credited with a therapeutic effect, such as angelica root, bletilla turnip, cyperus turnip, corydalis turnip, to name a few. The preparation of these products was fairly complicated and the use of numerous components raises the cost of that product, which excludes its mass production. Although honey was included in these products, there is no reference to an ability of the honey to provide a therapeutic effect in relation to the relief of stomach complaints.

Although compositions comprising honey and raw food fibers are known from WO 89/07399, GB 1571251, GB 821883 and FR 7731862, the specific therapeutic effects according to the present invention are not known therefrom. Furthermore, some of the afore mentioned publications use conditions such that the application of the high temperature has destroyed the specific effect of the active components.

The object of the present invention is to provide a composition to relieve GERD and stomach complaints, which composition resolves the above-mentioned disadvantages according to the prior art.

Another object of the present invention is to provide a composition to relieve GERD and stomach complaints, which, when used, can bring about the desired therapeutic effect within a short period of time.

Another object of the present invention is to provide a composition that has been processed into a compressed product.

Another object of the present invention is to provide a composition which normalizes the defective physiology of the digestion, and which is also a natural product without any side effects.

The present composition is characterized in that said honey has a peroxide activity amount of greater than 5 µg of hydrogen peroxide per gram honey after 60 minutes as measured at a temperature of 21 °C.

The relief mechanism of the composition according to the present invention in alleviating GERD and stomach complaints can only be partially explained. The composition of the present invention is consumed dry, (i.e. without water), whereby it is swallowed after having been chewed for as long as possible. The composition of the present invention fights indigestion, enhances the peristaltic movement, reduces the time it is in the intestines from start to finish by twenty to thirty percent, binds the bile acid and fights the stomach acid. Most users of the present composition reported that the main complaint, severe heartburn, disappears almost immediately. It is believed that the quick therapeutic effect is likely caused by the acid binding capability of the proteins in the saliva and the indirect stimulus of the peristaltic movement. The long-term changes in the digestive systems are attributed to the raw food fibers. Such raw food fibers modulate the digestion and promote a normal bowel movement, while the honey contributes to the protection of the mucosa. Stimulation of the saliva by intake or by chewing of the composition of the present invention for as long as possible helps the mucosa to recover. The saliva contains an epidermal growth factor whose purpose is to keep the mucosa healthy. Although the mechanism is not entirely understood, the honey component of the composition of the present invention contributes to the health of the mucosa.

The term "raw food fibers" as used herein includes food fibers which have not been modified, which means that heating and baking of these food fibers is excluded from the present invention, because such treatments would remarkably reduce the specific workings of these food fibers for the relief of GERD and stomach complaints. The raw food fibers according to the present invention are granular products, which have generally only been sieved and/or been cut so as to obtain the right size.

Although food products based on a combination of food fibers and honey are known, see for example DE 40 24 222, DE 43 22 421, WO 90/07880, CN 1096645 and CN 1100600, it is not known from these references that the specific combination of food fibers and honey promote the relief of GERD and stomach complaints. Moreover, food fibers that have gone through a baking process, as is the case in for example WO 90/07880, are expressly excluded from the raw food fibers defined in the composition of the present invention.

Preferably, the raw food fibers useful in the composition according to the present invention are wheat germ and/or wheat bran.

The raw food fibers, in particular the wheat germ and/or wheat bran, are to be included as a solid component, which, in combination with the honey, show a beneficial effect on the mucosa and the digestion. Wheat bran contains a substantial amount of phytic acid, whereby zinc and calcium are chelated. As for the wheat germs, these raw food fibers have minerals and vitamins and also have a pleasing taste. A combination of only honey and wheat bran has a low taste tolerance, while adding wheat germ to the composition can improve the tase to an "acceptable taste level". Another taste improving product is, for example, oat bran, which additionally has a positive effect of lowering the cholesterol amount in blood. Physiological experiments show different intragastric residence times for a fluid or for a fluid combined with dietary fibers. A fluid combined with dietary fibers remains in, for instance, the stomach for a longer residence time than a pure fluid will. Addition of a solid to the fluid, specifically the raw food fibers provided in this invention, increases the period of time that the composition including honey and raw food fibers remains in the stomach, so that there will be enough time for the composition to exercise its intended therapeutic effect.

The preference in the present composition would be, from a shelf-life point of view, that a non-denatured honey is used. Moreover, the honey has a peroxide activity amount of greater than about 5 µg of hydrogen peroxide per gram honey after 60 minutes as measured at a temperature of 21 °C. It would also be preferable, when taking the shelf-life of the honey into consideration, that the water content of that honey be less than about 17.5%.

Honey having the above properties is believed to be advantageous in order to maintain the honey's enzyme activity. It appears that the enzyme activity of the honey is an essential part of the composition. This enzyme activity disappears with heating or extended exposure to light. The shelf-life of the honey is an important parameter whereby honey with a water content greater than about 17.5% would not have a sufficiently long shelf-life. Hydrogen peroxide appears to influence cell division or proliferation of the mucosa, whereby the recovery of the mucosa depends on natural cell division or proliferation. Hence, cell damage and cell renewal should be in balance to keep the stomach mucosa healthy. Honey having a peroxide activity amount of greater than about 5 µg of hydrogen peroxide per gram honey after 60 minutes as measured at a temperature of 21 °C favours such as recovery of the mucosa. If the honey is heated, (e.g. through denaturation), the creation of hydrogen peroxide will be destroyed.

The present composition will preferably include 20-40 weight percent wheat bran, 20-40 weight percent wheat germ, and 20-60 weight percent honey, based on the total weight of the composition. The specific percentages are based on both taste appreciation and the therapeutic effect of the composition.

In addition, there could be one or more additives included in the composition of the present invention, including calcium carbonate, zinc oxide, pectin and carboxymethylcellulose. Moreover, it is also possible to add one or more taste improving additives to the composition.

The present invention further concerns processing or integrating the composition of the present invention into a compressed product. The term "compressed product" is to be understood to mean that the components of the present composition are formed into a homogenous entity. Baking and heating of these components is excluded because such processing would have a negative influence on the therapeutic effect sought after to relieve gastroesophageal reflux disease and stomach complaints. An example of such a compressed product includes a biscuit, cookie, bar and the like.

## Claims

1. The use of honey for the manufacture of a composition for gastroesophageal reflux disease and stomach complaints, wherein said composition further comprises raw food fibers, **characterized in that** said honey has a peroxide activity amount of greater than 5 µg of hydrogen peroxide per gram honey after 60 minutes as measured at a temperature of 21 °C.

2. The use of honey according to claim 1, **characterized in that** said raw food fibers further comprise wheat germ and/or wheat bran.

3. The use of honey according to claims 1-2, **characterized in that** said honey is a non-denatured honey.

4. The use of honey according to claims 1-3, **characterized in that** said honey has a water content of less than 17.5%.

5. A composition for the relief of gastroesophageal reflux disease and stomach complaints comprising honey and raw food fibers, **characterized in that** said honey has a peroxide activity amount of greater than 5 µg of hydrogen peroxide per gram honey after 60 minutes as measured at a temperature of 21 °C.

6. A composition according to claim 5, **characterized in that** said composition comprises 20-40 weight percent wheat bran, based on the total weight of the composition.

7. A composition according to claims 5-6 **characterized in that** said composition comprises 20-40 weight percent wheat germ, based on the total weight of the composition.

8. A composition according to claims 5-7, **characterized in that** said composition comprises 20-60 weight percent honey, based on the total weight of the composition.

9. A composition according to claims 5-8, **characterized in that** said composition comprises at least one additive selected from the group comprising calcium carbonate, zinc oxide, pectin and carboxymethylcellulose.

10. A compressed product comprising the composition according to claims 5-9.

## Patentansprüche

1. Verwendung von Honig zur Herstellung einer Zusammensetzung für gastroösophagealen Reflux und Störungen im Magenbereich, wobei die genannte Zusammensetzung weiter Rohfasern umfaßt, **dadurch gekennzeichnet, daß** der genannte Honig nach 60 Minuten bei einer Temperatur von 21 °C einen Peroxidwert von mehr als 5 µg Wasserstoffperoxid pro Gramm Honig hat.

2. Verwendung von Honig gemäß Anspruch 1, **dadurch gekennzeichnet, daß** die genannten Rohfasern weiter Weizenkeime und/oder Weizenkleie umfassen.

3. Verwendung von Honig gemäß Anspruch 1-2, **dadurch gekennzeichnet, daß** der genannte Honig ein nichtdenaturierter Honig ist.

4. Verwendung von Honig gemäß Ansprüchen 1-3, **dadurch gekennzeichnet, daß** der genannte Honig einen Wassergehalt von weniger als 17,5 % hat.

5. Zusammensetzung zur Verminderung von gastroösophagealem Reflux und Störungen im Magenbereich umfassend Honig und Rohfasern, **dadurch gekennzeichnet, daß** der genannte Honig nach 60 Minuten bei einer Temperatur von 21 °C einen Peroxidwert von mehr als 5 µg Wasserstoffperoxid pro Gramm Honig hat.

6. Zusammensetzung gemäß Anspruch 5, **dadurch gekennzeichnet , daß** die genannte Zusammensetzung auf der Grundlage des Gesamtgewichts der Zusammensetzung einen Massenanteil von 20-40 Prozent Weizenkleie hat.

7. Zusammensetzung gemäß Ansprüchen 5-6, **dadurch gekennzeichnet, daß** die genannte Zusammensetzung auf der Grundlage des Gesamtgewichts der Zusammensetzung einen Massenanteil von 20-40 Prozent Weizenkeimen hat.

8. Zusammensetzung gemäß Ansprüchen 5-7, **dadurch gekennzeichnet, daß** die genannte Zusammensetzung auf der Grundlage des Gesamtgewichts der Zusammensetzung einen Massenanteil von 20-60 Prozent Honig hat.

9. Zusammensetzung gemäß Ansprüchen 5-8, **dadurch gekennzeichnet, daß** die genannte Zusammensetzung mindestens einen Zusatzstoff aus der Gruppe Kalziumkarbonat, Zinkoxid, Pektin und Carboxymethylcellulose umfaßt.

10. Preßerzeugnis, umfassend die Zusammensetzung gemäß Ansprüchen 5-9.

## Revendications

1. Utilisation de miel pour la fabrication d'une composition pour soulager des symptômes de reflux gastro-oesophagien et de troubles gastriques, pour laquelle ladite composition, en outre, comprend des fibres alimentaires brutes, **caractérisée en ce que** ledit miel a un taux d'activité de peroxyde supérieur à 5 µg de peroxyde d'hydrogène par gramme de miel après 60 minutes, mesuré à une température de 21 °C.

2. Utilisation de miel selon la revendication 1, **caractérisée en ce que** lesdites fibres alimentaires brutes, en outre, comprennent du germe de blé et / ou du son de blé.

3. Utilisation de miel selon les revendications 1 à 2, **caractérisée en ce que** ledit miel est un miel non-dénaturé.

4. Utilisation de miel selon les revendications 1 à 3, **caractérisée en ce que** ledit miel a une teneur en eau inférieur à 17,5 %.

5. Composition pour soulager des symptômes de reflux gastro-oesophagien et de troubles gastriques, comprenant du miel et des fibres alimentaires brutes, **caractérisée en ce que** ledit miel a un taux d'activité de peroxyde supérieur à 5 µg de peroxyde d'hydrogène par gramme de miel après 60 minutes, mesuré à une température de 21 °C.

6. Composition selon la revendication 5, **caractérisée en ce que** ladite composition comprend de 20 à 40 % en masse de son de blé, relativement à la masse totale de la composition.

7. Composition selon la revendication 5 ou 6, **caractérisée en ce que** ladite composition comprend de 20 à 40 % en masse de germe de blé, relativement à la masse totale de la composition.

8. Composition selon les revendications 5 à 7, **caractérisée en ce que** ladite composition comprend de 20 à 60 % en masse de miel, relativement à la masse totale de la composition.

9. Composition selon les revendications 5 à 8, **caractérisée en ce que** ladite composition comprend au moins un additif sélectionné parmi le groupe comprenant un carbonate de calcium, un oxyde de zinc, une pectine et une carboxyméthylcellulose.

10. Produit comprimé comprenant la composition selon les revendications 5 à 9.
